# EUROPEAN PATENT APPLICATION

(11) **EP 1 818 065 A2**
(43) Date of publication of application: **15.08.2007**
(21) Application number: 07002454.2
(22) Date of filing: 05.02.2007
(51) Int. Cl.: A61L 15/58, A61L 24/04

(54) **Formulation for improving skin adhesive effectiveness**

(30) Priority: 08.02.2006 US 771357 P
(71) Applicant: Bristol-Myers Squibb Company, New York, N.Y. 10154 (US)
(72) Inventor: Badejo, Ibraheem T., North Carolina 27614 (US); Johnsen, Kenneth A., New Jersey 08854 (US); Cline, John B., New Jersey 08901 (US)
(74) Representative: Holmes, Miles Keeton

(57) **Abstract**

A cyanoacrylate monomer is used as an adhesion-enhancement material for enhancing at least an initial bond between a person's skin and a hydrocolloid adhesive of e.g., an ostomy or wound care appliance, for attachment to the skin. The monomer is applied to the skin in a non-activated state. Polymerization of the momoner is initiated by a material in the hydrocolloid adhesive when the adhesive appliance is pressed on the skin thereafter. The adhesion-enhancement material may further comprise a plasticizer having a low Tg (e.g., <10°C).

## Description

### FIELD OF THE INVENTION

The present invention relates to the field of skin adhesives, for example, for attaching a product to a wearer's skin. The invention is especially useful in the fields of wound care and ostomy, but the invention is not limited to these fields of use. In one non-limiting aspect, the invention may relate to a skin preparation material in combination with a skin adhesive.

### BACKGROUND TO THE INVENTION

Many ostomy and wound care products use skin-friendly hydrocolloid adhesives to bond to the user's skin. Hydrocolloid adhesives, generally, provide an excellent bond with the skin while protecting the skin from exposure to effluent. The hydrocolloid adhesive can also be peeled from the skin after use, leaving little or no residue on the skin. However, the bond strength between the hydrocolloid adhesive and the skin is relatively weak when the hydrocolloid adhesive is first applied, and may only reach its ultimate strength after up to 60 minutes of contact. The bond strength typically develops progressively during the first 15-60 minutes of wear. During the initial period, the developing bond may be undermined by contact with the effluent, reducing the performance of the adhesive in terms of the bond strength and the duration for which the adhesive is effective. This can lead to undesirably short wear times, and to a risk of leakage of effluent, causing potential discomfort and embarrassment for the wearer.

Although not relevant to the field of hydrocolloid adhesives, a skin adhesive is known in the form of 2-octylcyanoacrylate containing paraben. The skin adhesive is a liquid which is applied directly to the skin by using a special applicator containing an activator for polymerising the adhesive. Use of the applicator is essential for initiating the polymerization in order to cure the adhesive. Once the liquid has made contact with the activator on the applicator, the adhesive begins to cure quickly, and must be applied to the skin immediately. Such an adhesive technique is effective for sealing cuts and abrasions, but is totally impractical as an alternative to a conventional hydrocolloid adhesive, for attaching a device such as an ostomy appliance to the skin. The rapid curing of the liquid after application using the special applicator means that considerable dexterity would be needed to (i) apply the liquid quickly and accurately to the desired skin area using the special applicator, and then (ii) apply ostomy device quickly in the correct position before the adhesive cures. Such dexterity is completely beyond the ability of many ostomates (who are often elderly and/or infirm). Also, the difficulty of use would be off-putting in the extreme even for dexterous ostomates.

### SUMMARY OF THE INVENTION

The present invention has been devised bearing the above problems in mind.

One aspect of the present invention provides an adhesion-enhancement material for use with an adhesive body appliance. The adhesion-enhancement material provides one or more of the following features:
(a) enhancement of bonding (e.g., initial bonding) of the adhesive appliance to the skin;
(b) be a hydrocolloid adhesive;
(c) act, or be used, as an interface between the skin and the adhesive of the adhesive appliance;
(d) application to the skin as a skin preparation material or the surface of an adhesive applicance before an adhesive appliance is applied to the skin;
(e) a monomer, such as a cyanoacrylate, for example, an α-cyanoacrylate;
(f) application (e.g., either to the user's skin, or to the adhesive of the adhesive appliance) without using, during the application process, any additional activator for initiating polymerization of the monomer;
(g) polymerization is initiated automatically when the adhesion-enhancement material is brought into contact with the adhesive,for example, if the adhesion-enhancement material is initially applied to the skin, polymerization is initiated when the adhesive of the adhesive appliance is pressed onto the adhesion-enhancement material on the skin;
(h) polymerization is initiated by a material contained in the adhesive, for example, the monomer is configured to be initiated by weak nucleophiles or weak basic compounds, which may be in the adhesive;
(i) a reactive plasticizer that copolymerizes with the monomer to form a copolymer,or a non-reactive plasticizer that remains distinct from the polycyanoacrylate, having a relatively low Tg, so as to provide the adhesion-enhancement material with a degree of flexibility, even after polymerization;
(j) an initial bond(s) between the skin and the polymerized adhesion-enhancement material, and between the polymerized adhesion-enhancement material and the adhesive, that is stronger than the initial bond directly between the adhesive and the skin;
(k) a bond of the adhesive that transfers from bonding with the adhesion-enhancement material, to bonding with the underlying skin; and
(l) an adhesion-enhancement material that remains substantially intact with the adhesive, when peeled from the skin, so that the adhesion-enhancement material does not create substantial additional residue on the skin.

Viewed in another aspect, a cyanoacrylate monomer is used as an adhesion-enhancement material for enhancing at least an initial bond between a person's skin and a hydrocolloid adhesive of, e.g., an ostomy or wound care appliance, for attachment to the skin. The monomer is applied to the skin in a non-activated state. Polymerization of the monomer is initiated by a material in the hydrocolloid adhesive when the adhesive appliance is pressed on the skin thereafter. The adhesion-enhancement material may further comprise a reactive or non-reactive plasticizer having a low Tg (e.g., <10°C).

Other features of the invention are defined in the claims and/or will be apparent to one skilled in the art.

### BRIEF DESCRIPTION OF THE DRAWINGS

Fig. 1 is a flow-diagram of the steps of a first example for attaching an adhesive appliance to skin.
Fig. 2 is a schematic cross-section of an adhesion-enhancement material applied to a person's peristomal skin as a skin preparation material in accordance with the first example.
Fig. 3 is a schematic cross-section of an adhesive portion of an adhesive appliance applied to the skin in accordance with the first example.
Fig. 4 is a flow-diagram of the steps of a second example for attaching an adhesive appliance to skin.

### DETAILED DESCRIPTION OF THE PREFERRED EMBODIMENTS

Referring to the drawings, an adhesion-enhancement material 10 is illustrated for enhancing adhesion between a person's skin 12, and an adhesive 18 of an adhesive appliance 14. The adhesion-enhancement material 10 is provided or used as an interface between the skin 12 and the adhesive 18.

The adhesive appliance 14 may, for example, be an ostomy appliance, such as an ostomy pouch or a body-side coupling member for such a pouch. The skin 12 may be peristomal skin around a stoma 16 (indicated in phantom). Alternatively, the adhesive appliance 14 may be a wound care appliance, or some other appliance, such as a medical or non-medical appliance, or a personal hygiene appliance.

The adhesive appliance 14 comprises a layer or pad or wafer of the adhesive 18. The adhesive 18 is a skin-friendly adhesive. The adhesive 18 is a hydrocolloid-containing (e.g., hydrocolloid-based) adhesive. The adhesive 18 may, for example, be Stomahesive ^{®} or Durahesive ^{®} available from ConvaTec, a division of Bristol-Myers Squibb Company.

The adhesion-enhancement material 10 comprises a monomer (at least prior to activation after application of the adhesion-enhancement material). The monomer is preferably applied in a non-activated state (e.g., without using any additional activator during application of the adhesion-enhancement material 10). The monomer is preferably of a type that can be initiated by a material contained in the adhesive 18. For example, polymerization of the monomer may be initiated by weak nucleophiles or weak basic compounds. The activator material in the adhesive 18 may be a normal component of the adhesive composition (for example, sodium carboxymethyl cellulose (NaCMC)), or it may be a material added to the adhesive 18 solely to serve as an activator of the adhesion-enhancement material 10. The monomer is, preferably, of a type that can be initiated at ambient conditions, e.g., at room temperature or at normal skin-surface temperature. While the above is the preferred method, it is also possible to apply an initiator to the skin surface prior to the application of the monomer.

The monomer may be a 1,1-distributed ethylene monomer. The monomer may be of the formula: 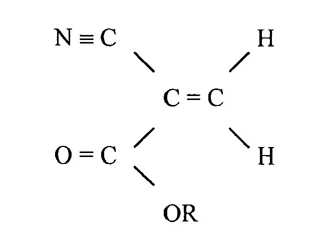 where R is selected from one or more of:
(i) alkyl groups having at least 1, and, preferably, not more than 20, carbon atoms;
(ii) hydrocarbyl or substituted hydrocarbyl groups, including straight chain or branched chain alkly groups having 1-20 carbon atoms;
(iii) straight chain or branched chain C₁-C₂₀ alkyl groups substituted with one or more of an acyloxy group, a haloalkyl group, an alkoxy group, a halogen atom, or a cyano group;
(iv) straight chain or branched chain alkenyl groups having 2 to 20 carbon atoms;
(v) straight chain or branched chain alkynyl groups having 2 to 12 carbon atoms;
(vi) cycloalkyl groups;
(vii) aralkyl groups;
(viii) alkylaryl groups;
(ix) aryl groups;
(x) alkylene alkoxy substituted hydrocarbons moiety having 1-8 carbon atoms; and
(xi) alkyl esters, having alkyl and alylene moities of 1-8 carbons, alkylene having 1-2 carbons.

The monomer may be a cyanoacrylate, for example, a polycyanoacrylate. The monomer may, for example, be any of: 2-octylcyanoacrylate, decyl cyanoacrylate, 2-ethylhexyl cyanacrylate, butyl cyanoacrylate, methyl cyanoacrylate, 3-methoxybutyl cyanoacrylate, 2-butoxyethyl cyanoacrylate, 2-isopropoxyethyl cyanoacrylate, 1-mthoxy-2-propyl cyanoacrylate.

The adhesion-enhancement material 10 further comprises a plasticizer. The plasticizer improves the flexibility of the adhesion-enhancement material 10 after polymerization. The plasticizer has a low Tg. For example, Tg is less than 20°C, preferably, less than 10°C. The lower the value of Tg, the less brittle the adhesion-enhancement material 10 (e.g., after polymerization). For attaching the adhesive appliance 14 to the skin 12, it is desired that the adhesive 18 and the layer of adhesion-enhancement material 10 have sufficient flexibility to conform to the skin 12. It is also desirable that the adhesion-enhancement material 10 and the adhesive 18 be capable of flexing to follow body movements or other changes of shape of the skin 12. This ensures good contact between the skin 12 and the combination of the skin preparation layer 10 and the adhesive 18 throughout the wear time of the adhesive appliance 14. Plasticizers may include one (or a combination of two or more) selected from: siloxanes, citrates and end capped polyethylene glycol.

Examples of two different uses of the adhesion-enhancement material 10 are now described. In the first example, the adhesion-enhancement material 10 is applied as a skin preparation material directly to the skin 12 before the adhesive 18 is subsequently pressed on to the skin 12 to attach the adhesive appliance 14. In the second example, the adhesion-enhancement material 10 is applied to the adhesive surface of the adhesive 18, whereafter the adhesive 18 is pressed on to the skin 12 to attach the adhesive appliance 14.

In either example, the adhesion-enhancement material 10 may be in liquid form, or a paste or gel. The skin preparation material 10 may be supplied and/or applied in any suitable form, for example: using an application swab; a spray bottle; a dropper bottle; a roll-on bottle; in a foil packet with an applicator wipe; in a breakable ampoule; in a bottle with a brush applicator; in a dispenser foil packet; in a felt-tip marker; in a paste tube; in a single-use breakable container (e.g., of plastics). The adhesion-enhancement material 10 may be substantially transparent (colorless) or it may be lightly or heavily colored as a visual aid. The adhesion-enhancement material 10 may be applied as a thin layer or coating, for example, having a thickness less than that of the adhesive 18. Such a thin coating may allow parallel bonds to develop as (i) a direct bond between the adhesive 18 and the skin 12, and (ii) an indirect (e.g., multi-stage) bond between the adhesive 18 and the adhesion-enhancement material 10 as an intermediate, and between the adhesion-enhancement material 10 and the skin 12.

In an alternative form, the adhesion-enhancement material 10 may be in the form of, or supported on, a discrete member (not shown) instead of being applied as a liquid, paste or gel.

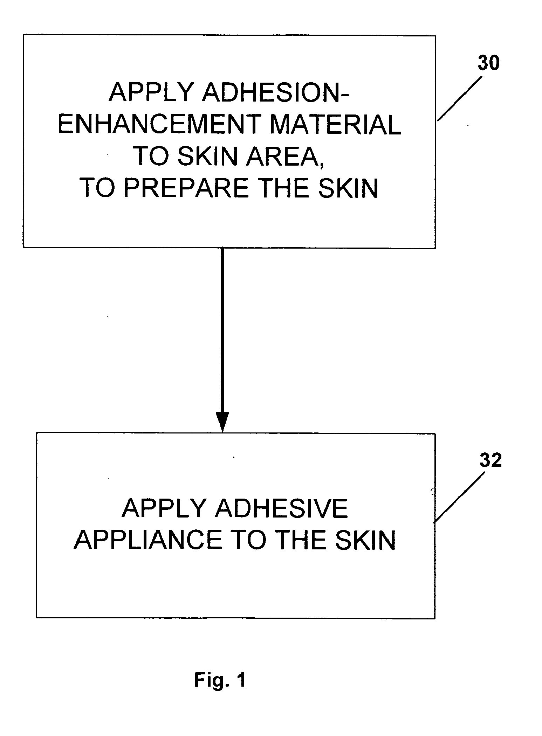

Referring to Figs. 1-3, in use, as a first step 30 prior to fitting the adhesive appliance 14, the user applies a layer of the adhesion-enhancement material 10 as a skin preparation material to the region of the skin 12 to which the adhesive appliance 14 will be fitted. The adhesion-enhancement material 10 may be applied without any activator, so that the monomer may remain unactivated (unpolymerized) at least while the adhesive-enhancement material 10 remains isolated on the skin 12 surface. This enables the adhesion-enhancement material 10 to be applied leisurely, and also allows the adhesion-enhancement material 10 to be easily spreadable over the desired area of the skin 12. Such a technique also avoids any need for the user to fit the adhesive appliance 14 immediately.

In a next step 32 (Fig. 1), after the user is satisfied with the coverage and distribution of the adhesion-enhancement material 10, the user then presses the adhesive 18 of the adhesive appliance 14 onto the target region of the skin 12. The adhesive surface of the wafer 18 may initially be covered by a protective sheet (e.g., of silicone release paper (not shown)) which may be removed to expose the adhesive surface of the wafer 18.

The combination of the adhesion-enhancement material 10 and the adhesive appliance 14 may be configured to achieve one or more of the following:
(a) an adhesion-enhancement material 10 containing a monomer is applied without a polymerization activator, so that the adhesion-enhancement material 10 may be applied in a non-activated state;
(b) a setting rate of the adhesion-enhancement material 10 sufficiently long that it can wet the skin 12 and the adhesive 18 when the adhesive 18 is pressed over the adhesion-enhancement material 10; . providing excellent contact between the skin 12, the adhesion-enhancement material 10 and the adhesive 18.
(c) polymerization of the monomer initiated by one or more components or ingredients of the adhesive 18 of the adhesive appliance 14, initiated automatically, at ambient conditions, upon pressing of the adhesive 18 of the adhesive appliance 14 into contact with the coating of the adhesion-enhancement material 10, thus avoiding the need for any additional activator to be used by the user;
(d) upon polymerization, the combination of the bond between the adhesion-enhancement material 10 and the skin 12, and between the adhesion-enhancement material 10 and the adhesive 18, that provides a stronger bond than the initial bond between the adhesive 18 and the skin 12 directly, enabling the initial bond strength of the adhesive 18 to be improved significantly, compared to applying the adhesive 18 directly to the skin 12 without using the adhesion-enhancement material 10;
(e) protection of the skin 12, and the underside of the adhesive 18, from corrosive effluent, even during initial development of a direct bond between the adhesive 18 and the skin 12;
(f) flexibility (e.g., by virtue of the plasticizer), to allow excellent conformability of the adhesive appliance 14 to the underlying skin 12.
(g) breathability (e.g., it allows moisture transmission from the skin 12 to the adhesive 18);
(h) a bond strength directly between the adhesive 18 and the underlying skin 12 that develops in a manner similar to a conventional hydrocolloid adhesive;
(i) a bond strength between the adhesion-enhancement material 10 and the skin 12 that deteriorates more slowly than the bond strength between the adhesion-enhancement material 10 and the adhesive 18.
(j) abond strength that is transferred to lie primarily in the direct bond developed between the adhesive 18 and the skin 12, rather than between the indirect bond provided by the adhesion enhancement material 10 and the skin 12; and
(k) an adhesive enhancement material 10 that remains intact with the adhesive 18, when the adhesive appliance 14 is peeled from the skin 12, leaving little or no residue on the skin 12.

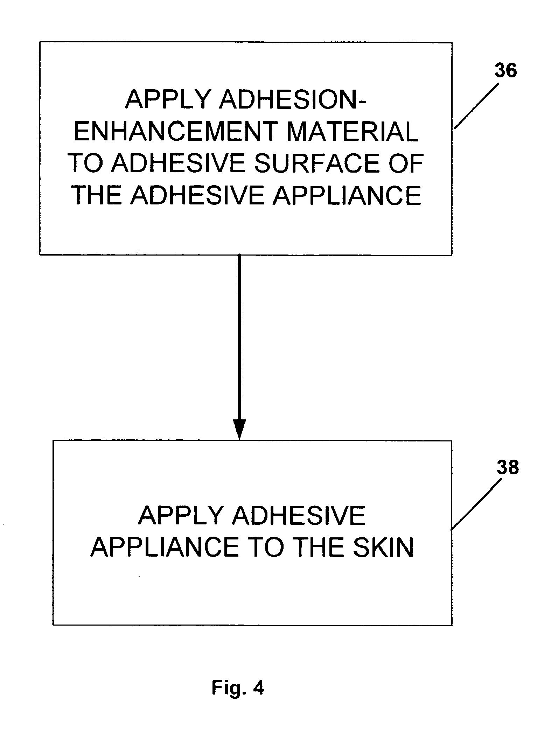

Referring to Fig. 4, the second example may now be described in more detail. In the second example, instead of applying the adhesion-enhancement material 10 directly to the skin, a first step 36 may be to apply the adhesion-enhancement material 10 to the adhesive surface of the adhesive 18. As mentioned above, the surface of the adhesive 18 may initially be protected by a sheet (not shown) intended to be removed before the adhesion-enhancement material 10 is applied to the adhesive 18.

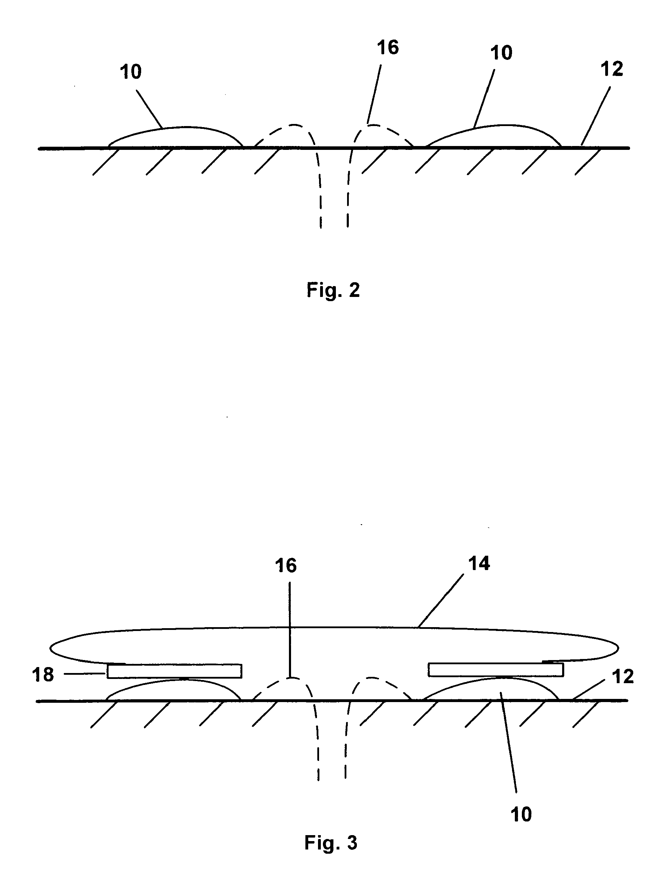

Thereafter, as a second step 38, the adhesive 18 bearing the adhesion-enhancement material 10 is pressed against the skin 12, so that the adhesion-enhancement material 10 provides an interface between the adhesive 18 and the skin 12 (Fig. 3).

In the second example, polymerization of the adhesion-enhancement material 10 is initiated when the adhesion-enhancement material 10 is applied to the surface of the adhesive 18. Therefore, the user is obliged to fit the adhesive appliance 14 to the skin 12 relatively quickly after applying the adhesion-enhancement material 10 to the adhesive 18. Nevertheless, the steps of the second example may still be carried out more leisurely than were the adhesion-enhancement material 10 to be applied initially to the skin 12 in an activated state. Also, the second example enables all of the features and advantages (a) and (c)-(k) of the first example to be achieved equally well. Alternatively, polymerization may be delayed (or at least slowed) until the adhesive 18 and the adhesion-enhancement material 10 are warmed to body temperature when pressed against the skin 12.

The foregoing description is merely illustrative of a preferred embodiment of the invention. Many modifications and equivalents may be used within the scope and/or sprit of the invention. In particular, it will be appreciated that the invention is not limited only to the specific materials described above, and that some or all of the above effects may be achieved using other cyanoacrylate or non-cyanoacrylate materials, based on the same principles explained above. Nevertheless, a cyanoacrylate remains as a highly preferred monomer for the adhesion-enhancement material in view of its excellent bonding strength.

## Claims

1. An adhesion-enhancement material for enhancing the adhesion between a person's skin and a skin-friendly adhesive of an adhesive appliance, wherein the adhesion-enhancement material comprises a monomer for application without a polymerization activator, to serve as an interface material between the adhesive appliance and the person's skin, and wherein the monomer is configured to be initiated by a material contained in the adhesive appliance.

2. The adhesion-enhancement material of claim 1, wherein the adhesion-enhancement material is configured to be applied without a polymerization activator directly to the person's skin.

3. The adhesion-enhancement material of claim 1, wherein the adhesion-enhancement material is configured to be applied without a polymerization activator directly to an adhesive surface of the adhesive appliance.

4. The adhesion-enhancement material of claim 1, 2 or 3, wherein the adhesion-enhancement material is configured to be initiated by a weak nucleophile and/or by a weak basic compound.

5. The adhesion-enhancement material of any preceding claim, wherein the monomer is a 1,1-disustituted ethylene monomer.

6. The adhesion-enhancement material of claim 5, wherein the 1,1-disubstituted ethylene monomer is a cyanoacrylate monomer.

7. The adhesion-enhancement material of claim 6, wherein the cyanoacrylate monomer has the formula: 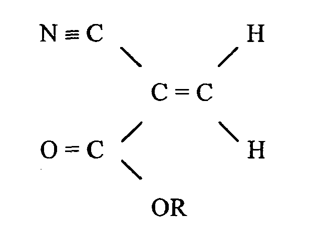 where R is selected from one or more of the following:
(i) alkyl groups having from 1 to 20 carbon atoms;
(ii) hydrocarbyl and/or substituted hydrocarbyl groups, including straight chain or branched chain alkly groups having 1-20 carbon atoms;
(iii) straight chain or branched chain C₁-C₂₀ alkyl groups substituted with one or more of an acyloxy group, a haloalkyl group, an alkoxy group, a halogen atom, a cyano group, or a haloalkyl group;
(iv) straight chain or branched chain alkenyl groups having 2 to 20 carbon atoms;
(v) straight chain or branched chain alkynyl groups having 2 to 12 carbon atoms;
(vi) cycloalkyl groups;
(vii) aryl groups;
(viii) alkylaryl groups;
(ix) alkylene alkoxy substituted hydrocarbons moiety having 1-8 carbon atoms; and
(x) alkyl esters, having alkyl and alylene moities of 1-8 carbons, alkylene having 1-2 carbons.

8. The adhesion-enhancement material of any preceding claim, wherein the adhesion-enhancement material further comprises a plasticizer.

9. The adhesion-enhancement material of claim 8, wherein the plasticizer has a low Tg.

10. The adhesion-enhancement material of claim 8 or 9, wherein the plasticizer has a Tg less than about 10°C.

11. The adhesion-enhancement material of claim 8, 9 or 10, wherein the plasticizer comprises at least one material selected from siloxane, citrate, and end-capped polyethylene glycol.

12. The adhesion-enhancement material of any preceding claim, wherein the adhesion-enhancement material is configured for use with a hydrocolloid adhesive.

13. The adhesion-enhancement material of any preceding claim, wherein the adhesion-enhancement material is configured such that an initial bond strength between the skin and the adhesion-enhancement material and between the adhesion-enhancement material and the adhesive, is greater than an initial bond strength between the adhesive and the skin.

14. The adhesion-enhancement material of any preceding claim, wherein the adhesion-enhancement material is configured such that a bond strength between the adhesion-enhancement material and the skin declines over a shorter period of time than does a bond strength between the adhesion-enhancement material and the adhesive.

15. An adhesion-enhancement material for enhancing a bond between skin and an adhesive appliance for attachment to the skin, the adhesion-enhancement material comprising:
a monomer; and
a plasticizer having a low Tg.

16. The adhesion-enhancement material of claim 15, wherein the monomer is initiated by a weak nucleophile

17. The adhesion-enhancement material of claim 15, wherein the monomer is initiated by a weak basic compound.

18. The adhesion-enhancement material of claim 15, 16 or 17, wherein the monomer is a 1,1-disubstituted ethylene monomer.

19. The adhesion-enhancement material of claim 18, wherein the 1,1-disubstituted ethylene monomer is a cyanoacrylate monomer.

20. The adhesion-enhancement material of claim 19, wherein the cyanoacrylate monomer has the formula: 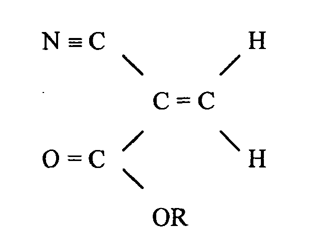 where R is selected from one or more of the following:
(i) alkyl groups having from 1 to 20 carbon atoms;
(ii) hydrocarbyl and/or substituted hydrocarbyl groups, including straight chain or branched chain alkly groups having 1-20 carbon atoms;
(iii) straight chain or branched chain C₁-C₂₀ alkyl groups substituted with one or more of an acyloxy group, a haloalkyl group, an alkoxy group, a halogen atom, or a cyano group;
(iv) straight chain or branched chain alkenyl groups having 2 to 20 carbon atoms;
(v) straight chain or branched chain alkynyl groups having 2 to 12 carbon atoms;
(vi) cycloalkyl groups;
(vii) aryl groups;
(viii) alkylaryl groups;
(ix) alkylene alkoxy substituted hydrocarbons moiety having 1-8 carbon atoms; and
(x) alkyl esters, having alkyl and alylene moities of 1-8 carbons, alkylene having 1-2 carbons.

21. The adhesion-enhancement material of claim 15 or any claim dependent thereon, wherein the plasticizer has a Tg less than about 10°C.

22. The adhesion-enhancement material of claim 15 or any claim dependent thereon, wherein the plasticizer comprises at least one material selected from siloxane, citrate, and end-capped polyethylene glycol.

23. A material comprising a skin-friendly adhesive material and an adhesion-enhancement material for contacting the adhesive material to enhance adhesion, wherein:
the adhesive material comprises a pressure sensitive adhesive and one item selected from: a monomer and an activator for the monomer;
the adhesion-enhancement material comprises the other item selected from said monomer and said activator for the monomer;
wherein the adhesive material and the adhesive-enhancement materials are separate materials, and polymerization of the monomer is initiated when the adhesive material and the adhesion-enhancement material are brought into contact with each other.

24. The material of claim 23, wherein the adhesive material comprises the activator, and the adhesion-enhancement material comprises the monomer.

25. The material of claim 23 or 24, wherein the activator comprises a weak nucleophile and/or a weak basic compound.

26. The material of claim 25, wherein the activator comprises a carboxymethylcellulose.

27. The material of claim 23 or any claim dependent thereon, wherein the adhesive material comprises a hydrocolloid adhesive.

28. The material of claim 23 or any claim dependent thereon, wherein the monomer is a 1,1-disubstituted ethylene monomer.

29. The material of claim 28, wherein the 1,1-disubstituted ethylene monomer is a cyanoacrylate monomer.

30. The material of claim 29, wherein the cyanoacrylate monomer has the formula: 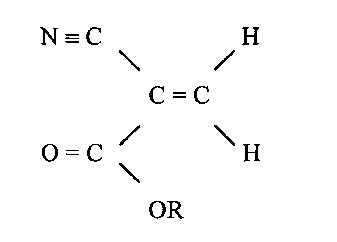 where R is selected from one or more of the following:
(i) alkyl groups having from 1 to 20 carbon atoms;
(ii) hydrocarbyl and/or substituted hydrocarbyl groups, including straight chain or branched chain alkly groups having 1-20 carbon atoms;
(iii) straight chain or branched chain C₁-C₂₀ alkyl groups substituted with one or more of an acyloxy group, a haloalkyl group, an alkoxy group, a halogen atom, or a cyano group;
(iv) straight chain or branched chain alkenyl groups having 2 to 20 carbon atoms;
(v) straight chain or branched chain alkynyl groups having 2 to 12 carbon atoms;
(vi) cycloalkyl groups;
(vii) aryll groups;
(viii) alkylaryl groups;
(ix) alkylene alkoxy substituted hydrocarbons moiety having 1-8 carbon atoms; and
(x) alkyl esters, having alkyl and alylene moities of 1-8 carbons, alkylene having 1-2 carbons.

31. The material of claim 23 or any claim dependent thereon, further comprising a plasticizer.

32. The material of claim 31, wherein the plasticizer is comprised in the same material as the monomer.

33. The material of claim 31, wherein the plasticizer is comprised in the adhesion-enhancement material.

34. The material of claim 31, 32 or 33, wherein the plasticizer has a low Tg.

35. The material of claim 31, 32, 33 or 34, wherein the plasticizer has a Tg in a range of from 0°C to not significantly more than 10°C.

36. The material of claim 31 or any claim dependent thereon, wherein the plasticizer comprises at least one material selected from: siloxane, citrate, and end-capped polyethylene glycol.

37. The material of claim 23 or any claim dependent thereon, wherein an initial bond strength between the skin and the polymerized monomer and between the polymerized monomer and the adhesive material, is greater than an initial bond strength between the adhesive material and the skin.

38. The material of claim 23 or any claim dependent thereon, wherein a bond strength between the polymerized monomer and the skin declines over a shorter period of time than does a bond strength between the polymerized monomer and the adhesive material.

39. The method of interfacing an adhesion-enhancement material between skin and an adhesive appliance to enhance at least an initial bond strength between the skin and the adhesive appliance, wherein the adhesion-enhancement material comprises a monomer polymerizable by a material contained in the adhesive appliance.

40. The method of claim 39, wherein the monomer is applied directly to the skin in a non-activated state.

41. The method of claim 40, wherein the adhesive appliance is applied over the monomer after the monomer is applied to the skin.

42. The method of claim 39, 40 or 41, wherein the adhesive appliance comprises a hydrocolloid adhesive.

43. A method of enhancing at least an initial bond strength between skin and adhesive appliance for attachment to the skin, the method comprising:
applying in a non-activated state a monomer polymerizable by a material contained in the adhesive appliance, the monomer being applied to serve as an interface between the skin and the adhesive appliance.

44. The method of claim 43, wherein the step of applying the monomer comprises applying the monomer directly to the skin.

45. The method of claim 44, further comprising the step of applying the adhesive appliance to the skin after the monomer has been applied to the skin.

46. A method of providing an attachment for attaching an adhesive appliance to a person's skin, the method comprising:
providing a monomer polymerizable to form a bond with the skin; and
providing said adhesive appliance comprising (i) a activator for polymerizing the monomer, and (ii) a pressure sensitive adhesive for bonding to the skin independently of the monomer;
whereby the attachment is provided by combining the monomer and the adhesive appliance in use.

47. A method for attaching an adhesive appliance to a person's skin, comprising:
providing a monomer polymerizable to form a bond with the skin;
providing said adhesive appliance comprising (i) an activator for polymerizing the monomer, and (ii) a pressure sensitive adhesive for bonding to the skin independently of the monomer; and
using the monomer with the adhesive appliance to attach the adhesive appliance to the skin.
